# EUROPEAN PATENT SPECIFICATION

(11) **EP 4 351 712 B1**
(45) Date of publication and mention of the grant of the patent: **11.02.2026**
(21) Application number: 22728645.7
(22) Date of filing: 25.05.2022
(51) Int. Cl.: A61N 1/37, A61N 1/39, A61N 1/372, G16H 40/63, A61N 1/375

(54) **METHOD FOR CONTROLLING THE OPERATION OF AT LEAST TWO IMPLANTABLE MEDICAL DEVICES**
VERFAHREN ZUR STEUERUNG DES BETRIEBS VON MINDESTENS ZWEI IMPLANTIERBAREN MEDIZINPRODUKTEN
MÉTHODE DE COMMANDE DE FONCTIONNEMENT D'AU MOINS DEUX DISPOSITIFS MÉDICAUX IMPLANTABLES

(30) Priority: 09.06.2021 EP 21178516
(43) Date of publication of application: 17.04.2024
(73) Proprietor: BIOTRONIK SE & Co. KG, 12359 Berlin (DE)
(72) Inventor: DÖRR, Thomas, 12737 Berlin (DE); MÜSSIG, Dirk, West Linn, Oregon 97068 (US)
(74) Representative: Biotronik Corporate Services SE
(86) International application number: PCT/EP2022/064221
(87) International publication number: WO 2022/258382

(56) References cited:
- US-A1- 2002 049 481
- US-A1- 2013 226 263
- US-A1- 2016 059 024
- US-A1- 2016 206 892

## Description

The present invention relates to a method for controlling the operation of at least two implantable medical devices for stimulating a human or animal heart, to a cardiac rhythm management system and to a computer program product.

Prior art describes various possibilities of monitoring the functioning of implantable medical devices. To give an example, the operational parameters of different devices are displayed on the same display. A user can then decide whether or not the displayed parameters may allow proper functioning of the medical devices.

US 2016/0059024 A1 describes a cardiac rhythm management system including a first implantable device such as a defibrillator and a second implantable device such as a leadless cardiac pacemaker. A programmer is configured to receive and display heart data emanating from the implantable defibrillator and from the leadless cardiac pacemaker. The heart data emanating from the leadless cardiac pacemaker is displayed in temporal alignment with the heart data emanating from the implantable defibrillator.

US 2011/0022981 A1 describes a graphical user interface displaying a first device profile and a second device profile, wherein the first and second device profiles each comprise at least one device parameter used to configure a medical device of a subject.

US 2007/0088405 A1 describes a biostimulator system comprising one or more implantable devices and an external programmer configured for communicating with the implantable device or devices via bidirectional communication pathways. The communication pathways comprise a receiving pathway that decodes information encoded on stimulation pulses generated by one of the implantable devices and conducted through body tissue to the external programmer.

In document US 2016/206892 A1 an external medical device is described which is capable of bidirectional wireless communication with multiple implantable medical devices (IMDs). The external medical device is configured to determine an active membership of an IMD system present in a patient, establish programmable parameters for the IMD system based on the active membership, and transmit values for the established programmable parameters to the active membership.

In document US 2002/049481 A1 a system and method of detecting and displaying parameter interactions is described. A medical device system has a medical device and a programming interface, wherein the medical device includes a plurality of features which can be programmed via the programming interface. A plurality of icons is defined, wherein the plurality of icons includes a first icon, a second icon and a third icon, wherein the first icon indicates correctness, wherein the second icon warns of a parameter interaction and wherein the third icon warns of impermissible parameter settings. Parameter interactions between feature parameters are identified and each of the parameter interactions is associated with one of the plurality of icons. A parameter value is accepted and examined to determine if it causes a parameter interaction. If the parameter value causes a parameter interaction, the icon associated with the parameter interaction is displayed.

In document US 2013/226263 A1 a system is described having a programming device in communication with an implantable medical device, an implantable sensor, and electronic medical records. A user interface is in communication with the programming device, and the user interface is configured to receive an override parameter and override rationale.

It is an object of the present invention to provide a method enabling an operation of at least two implantable medical devices that is safer than an operation according to prior art methods. It is another object of the present invention to provide a corresponding system allowing a safer operation than prior art systems do.

This object is achieved with a method for controlling the operation of at least two implantable medical devices for stimulating a human or animal heart having the claim elements of claim 1. Such a method comprises the steps explained in the following.

In one of the method steps, a first operational parameter of a first of the at least two implantable medical devices is received with a control device.

In another method step, a second operational parameter of a second device of the at least two implantable medical devices is received with the same control device.

Afterwards, the first operational parameter and the second operational parameter are checked against each other to identify a potential conflict precluding a safe operation of the first device and the second device. Thus, this checking step aims in identifying a parameter conflict between the first operational parameter and the second operational parameter.

If such a parameter conflict has been identified, the first operational parameter, the second operational parameter and data on the conflict are visualized on a display of the control device. The data on the conflict may have the form of plaintext (like a warning) and/or may include one or more icons drawing the attention of a user of the control device to the identified conflict. In an embodiment, the data may include a specific color used for displaying a parameter like the first operational parameter and/or the second operational parameter and/or text relating to the operation of the first implantable medical device and/or the second implantable medical device.

The claimed method enables an automatic identification of parameter conflicts occurring during operation of two or more implantable medical devices that impede a safe operation of the implantable medical devices. According to prior art solutions, such an identification of parameter conflicts is only manually possible and requires a deep knowledge as well as extended experience of medical staff being responsible for the implantation and/or operation of such implantable medical devices. The presently claimed invention overcomes this human factor and thus reduces the risk of an inappropriate operation of different medical device due to human errors.

In an embodiment, the control device receives the first operational parameter and/or the second operational parameter from an input of the user of the control device. Thus, upon programming the first implantable medical device and/or the second implantable medical device, the user typically chooses a set of parameters under which the operation of the respective medical device is to be performed. According to this embodiment, it is possible to directly check the parameters intended to be used for the operation of the implantable medical devices upon inputting the same.

In an embodiment, the control device receives the first operational parameter from a memory of the first implantable medical device and/or the second operational parameter from a memory of the second implantable medical device. In such a case, a compliance check of the first and second operational parameters is not directly performed during input of the parameters, but rather on the basis of an already performed programming of the first and/or second implantable medical device, i.e., on the basis of previously stored values. It is possible to combine the embodiment of checking at least one of the parameters upon inputting the same and at least one other of the parameters on the basis of a stored value. Such a combination offers high flexibility for a user of the control device and makes it possible to adapt the settings of one of the implantable medical devices without amending the settings of the other of the implantable medical devices, but nonetheless to take advantage of an automatic parameter conflict check between the first operational parameter of the first device and the second operational parameter of the second device.

In an embodiment, an operation of at least one of the implantable medical devices will only be allowed if no parameter conflict has been identified by the presently described method. This embodiment allows a particularly safe operation of the implantable medical devices since it automatically prevents an operation with undesired or inappropriate operational parameters.

In an embodiment, the first operational parameter is chosen from the group consisting of a stimulation frequency, in particular a stimulation frequency of an antibradycardic stimulation or a stimulation frequency of a post-shock stimulation, frequency boundaries that need to be exceeded for delivering an antitachycardic pacing, and a condition to be fulfilled for delivery of an antitachycardic pacing.

In an embodiment, the second operational parameter is chosen from the group consisting of a lower frequency boundary for defibrillation, an upper frequency boundary for defibrillation, a frequency zone for defibrillation between a lower and an upper frequency boundary, and an activation of a shock function of the second implantable medical device.

In an embodiment, at least one of the combinations of the first operational parameter and the second operational parameter listed in the following table is chosen for carrying out the method. In another embodiment, the condition indicated in the third column of the following table needs also to be fulfilled.

| **First operational parameter** | **Second operational parameter** | **Condition to be fulfilled (parameter conflict rule)** |
|---|---|---|
| Stimulation frequency | Lower frequency boundary for defibrillation | The stimulation frequency needs always to be smaller than the lower frequency boundary for defibrillation. |
| Stimulation frequency of an antibradycardic stimulation | Lower frequency boundary for defibrillation | The stimulation frequency of an antibradycardic stimulation needs always to be smaller than the lower frequency boundary for defibrillation. |
| Stimulation frequency of a post-shock stimulation | Lower frequency boundary for defibrillation | The stimulation frequency of a post-shock stimulation needs always to be smaller than the lower frequency boundary for defibrillation. |
| Frequency boundaries that need to be | Upper frequency boundary for | The frequency boundaries that need to be exceeded for delivering an antitachycardic |
| exceeded for delivering an antitachycardic pacing | defibrillation | pacing need always to be smaller than the upper frequency boundary for defibrillation. |
| Frequency boundaries that need to be exceeded for delivering an antitachycardic pacing | Frequency zone for defibrillation between lower and upper frequency boundary | If the frequency boundaries that need to be exceeded for delivering an antitachycardic pacing lie within the frequency zone for defibrillation between lower and upper frequency boundary, an antitachycardic pacing function can only be activated if a shock delay of the second medical device is activated. |
| Delivery of an antitachycardic pacing allowed? | Shock function activated? | The delivery of an antitachycardic pacing is only allowed if the shock function of the second implantable medical device is activated. |

In an embodiment, at least one of the implantable medical devices is an active implantable medical device.

The term "active implantable medical device" is well known to a person skilled in the art. An "active medical device" is typically defined to be any medical device relying for its functioning on a source of electrical energy or any source of power other than that directly generated by the human body or gravity.

An "active implantable medical device" is typically defined to be any active medical device which is intended to be totally or partially introduced, surgically or medically, into the human body or by medical intervention into a natural orifice, and which is intended to remain after the procedure.

Further details on active implantable medical devices can be found, e.g., in the consolidated text of the Council Directive 90/385/EEC of 20 June 1990 on the approximation of the laws of the Member States relating to active implantable medical devices with subsequent amendments in the version published on 11 October 2007. The consolidated text of this Council Directive is freely accessible under the following link: https://eur-lex.europa.eu/legal-content/EN/TXT/?uri=CELEX:01990L0385-20071011

In an embodiment, the active implantable medical device is an implantable pulse generator (IPG), an implantable leadless pacemaker (iLP), an implantable cardioverter-defibrillator (ICD), or a device for cardiac resynchronization therapy (CRT).

In an embodiment, the first implantable medical device is an implantable leadless pacemaker (iLP). Such a leadless pacemaker is typically implanted into the right ventricle of the patient's heart and enables an antitachycardic or an antibradycardic pacing of the heart into which it is implanted.

In an embodiment, the leadless pacemaker is particularly adapted for providing an antitachycardic stimulation of the human or animal heart into which it is implanted.

In an embodiment, the second device is an implantable cardioverter/defibrillator (ICD). A combination of an implantable leadless pacemaker as first device and an implantable cardioverter/defibrillator as second device is particularly appropriate.

In an embodiment, the second device is a non-transvenously implantable cardioverter/defibrillator. A cardioverter/defibrillator to be implanted in a subcutaneous or substernal manner is a particular appropriate example for such a non-transvenously implantable cardioverter/defibrillator. The combination of an implantable leadless pacemaker and a non-transvenously implantable cardioverter/defibrillator is a particular appropriate combination. In such a case, it is not necessary to guide any electrode through a vein of the patient. Rather, the implantable leadless pacemaker is implanted within a cardiac chamber, typically within the right ventricle, whereas an electrode of the cardioverter/defibrillator is located outside the heart. By such an implantation, no blood vessels of the patient are permanently stressed by implanted electrodes. Thus, such a combination of implantable medical devices ensures a particularly high physiologic compatibility.

In an embodiment, the first device comprises a first patient identifier and the second device comprises a second patient identifier. In this context, the control device interrogates the first patient identifier and the second patient identifier and only performs the checking of the presence of a parameter conflict between the first operational parameter and the second operational parameter if the first patient identifier and the second patient identifier are assigned to the same patient. In an embodiment, the first patient identifier and the second patient identifier are assigned to the same patient if both patient identifiers are identical. In an embodiment, data relating to an assignment of the patient identifiers to a specific patient is stored in an electronically available dataset such as a database or a data table. By such an additional checking of an assignment of the implantable medical devices to one and the same patient, it is ensured in a particularly appropriate way to only correlate the operational parameters of those implantable medical devices that indeed influence each other since they are used for treating the same patient. A parameter conflict between operational parameters of implantable medical devices being implanted to different patients typically does not exist or, at least, is not physiologically relevant.

In an embodiment, the method is carried out by a programming device application and/or a server application and/or an application for remote monitoring the at least two implantable medical devices and/or an application for remote programming the at least two implantable medical devices and/or a mobile app. The use of a dedicated programming device for programming the at least two implantable medical devices is a particular appropriate implementation for carrying out the presently claimed method. The execution of the presently claimed method by a mobile app that can be activated on any generic mobile device such as a smartphone or a tablet is also a particularly appropriate implementation of the presently claimed method.

The present invention provides a cardiac rhythm management system as defined in claim 13.

Such a system comprises a first implantable medical device for stimulating a human or animal heart, a second implantable medical device for stimulating the same heart, and a control device that is operatively coupled to the first implantable medical device and the second implantable medical device. In this context, the control device comprises a memory and a processor. The memory comprises computer-readable code that causes the processor to perform the steps explained in the following when executed on the processor.

In one of the method steps, a first operational parameter of the first implantable medical device is received with the control device.

In another method step, a second operational parameter of the second implantable medical device is received with the control device.

Afterwards, it is automatically checked whether there exists a conflict between the first operational parameter and the second operational parameter, wherein the conflict would preclude a safe operation of the first implantable medical device and the second implantable medical device.

If such a parameter conflict has been identified, the first operational parameter, the second operational parameter, and data relating to the conflict are visualized on a display of the control device.

In an embodiment, the control device is operatively coupled to the first implantable medical device and to the second implantable medical device in a wireless manner. All standard data transmission protocols or specifications are appropriate for such a wireless data communication. Examples of standard data transmission protocols or specifications are the Medical Device Radiocommunications Service (MICS), the Bluetooth Low Energy (BLE) protocol and the Zigbee specification.

The present invention also provides a computer program product as defined in claim 15.

In one of the method steps, a first operational parameter of a first implantable medical device is received.

In another method step, a second operational parameter of a second implantable medical device is received.

Afterwards, it is automatically checked whether there exists a conflict between the first operational parameter and the second operational parameter, wherein the conflict would preclude safe operation of the first implantable medical device and the second implantable medical device.

If such conflict has been identified, the first operational parameter, the second operational parameter and data on the conflict are visualized on a display. Such a computer program product is a particularly appropriate implementation of the method described above.

The present invention is defined by the appended claims.

Further details of aspects of the present invention will be explained in the following making reference to exemplary embodiments and accompanying Figures. In the Figures:
- Figure 1: shows an embodiment of a cardiac rhythm management system;
- Figure 2: shows an embodiment of a control device with a display; and
- Figure 3: shows an embodiment of a method for controlling the operation of two implantable medical devices.

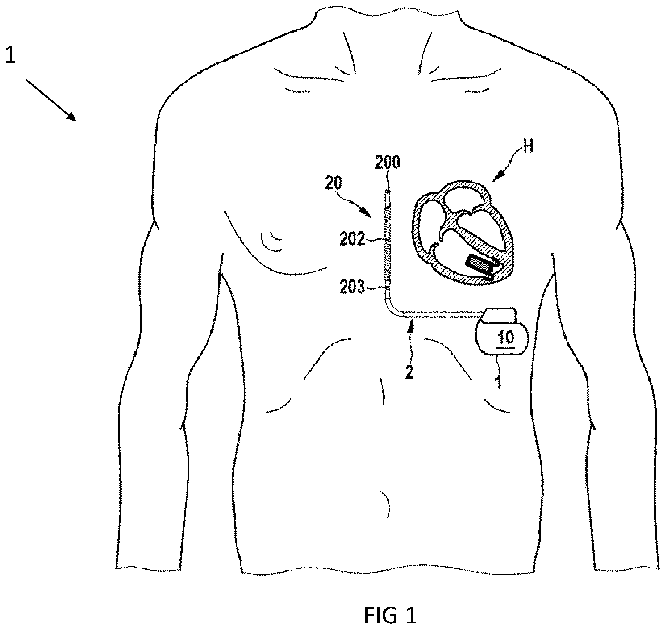

Figure 1 schematically depicts the torso of a human patient 1 including a heart 2 of the patient 1. An implantable leadless pacemaker (iLP) 3 is implanted into the right ventricle 4 of the heart 2. The iLP 3 represents a first implantable medical device for stimulating the human heart 2.

The patient 1 further carries a subcutaneous implantable cardioverter/defibrillator (ICD) 5. This ICD 5 serves as second implantable medical device for stimulating the human heart 2. It comprises an ICD pulse generator 6 within a housing 7 as well as an electrode lead 8 connected to the ICD pulse generator 6. The electrode lead 8 comprises a tip electrode 9 and a ring electrode 10, both serving as detection or sensing electrodes of the ICD 5. A shock coil 11 is located between the tip electrode 9 and the ring electrode 10. This shock coil 11 is substernally implanted next to and outside of the heart 2 of the patient 1. It serves for applying an electric shock to the heart 2 in case that defibrillation of the heart 2 is required. The necessary energy for this shock is generated by the ICD pulse generator 6. Thus, the ICD 5 serves for restoring regular heart function after a severe cardiac fibrillation.

In contrast, the iLP 3 is intended to treat a ventricular tachycardia (a less severe cardiac state than a fibrillation) and to stimulate the heart 2 with an antitachycardic pacing. The general functional range of the combination of the iLP 3 and the ICD 5 is comparable to that of a transvenously implanted cardioverter/defibrillator.

A plurality of operational parameters of the iLP 3 and of the ICD 5 can be programmed to adjust the functionality of the iLP 3 and the ICD 5 to the physiological needs of the patient 1. To give an example, the operational parameters of the ICD 5 comprise zone boundaries for classification of a tachycardia as requiring treatment. If a lower zone boundary is exceeded, this indicates the presence of a ventricular tachycardia (VT). If an upper zone boundary is exceeded, this indicates ventricular fibrillation (VF). Further operational parameters of the ICD 5 comprise, e.g., appropriate therapies for adjusting the physiologic state of the patient 1 if the heartbeat of the patient 1 exceeds one of the zone boundaries.

An operational parameter of the iLP 3 to be set is the stimulation frequency employed during cardiac pacing.

In order to allow a correct functioning of the iLP 3 and the ICD 5, it is mandatory that the iLP 3 does not stimulate the heart 2 with a higher frequency than the frequency chosen as lower boundary of the ICD 5 to detect a tachycardia. In this example, the applied parameter conflict rule would be that the stimulation rate of the iLP 3 needs always to be lower than the lower zone boundary of the ICD 5.

In the following, a further example will be explained illustrating another concrete mode of operation of the iLP 3 and the ICD 5.

In this example, the iLP 3 is intended to deliver an antibradycardic pacing, a post-shock stimulation and/or an antitachycardic pacing, as needed by the patient 1. The decision for delivering an antitachycardic pacing is made by the iLP 3, i.e., the iLP 3 comprises an own detection unit for detecting a tachycardia as well as two programmable frequency boundaries for the antitachycardic therapy to be delivered.

The ICD 5 is designed to provide a shock therapy if a cardiac rate lying above a frequency boundary is detected. In a first frequency zone (cardiac rate lying between a lower zone boundary and an upper zone boundary), the ICD 5 initially awaits an antitachycardic pacing therapy delivery by the iLP 3, if an according shock delay parameter is set in the ICD 5. In a second frequency zone (cardiac rate lying above the upper zone boundary), the shock therapy will always be delivered without waiting for an antitachycardic pacing by the iLP 3.

For this setup, the following parameter conflict rules need to be regarded when operating both the iLP 3 and the ICD 5 in a safe manner:
1) The stimulation frequency of the antibradycardic stimulation by the iLP 3 must always be smaller than the lower frequency boundary of the ICD 5.
2) The stimulation frequency of the post-shock stimulation of the iLP 3 must always be smaller than the lower frequency boundary of the ICD 5.
3) The frequency boundaries that need to be exceeded for delivering an antitachycardic pacing by the iLP 3 must always be smaller than the higher frequency boundary of the ICD 5.
4) If one or both of the frequency boundaries of the iLP 3 that need to be exceeded for delivering an antitachycardic pacing lies within the first frequency zone of the ICD 5, the delivery of the antitachycardic pacing of the iLP 3 can only be activated if a shock delay of the ICD 5 is activated.
5) The delivery of an antitachycardic pacing of the iLP 3 can only be activated if the shock function of the ICD 5 is activated.

It is apparent that the precedingly explained exemplary embodiment can also be applied by other implantable medical devices for stimulating the heart. Thus, it is not necessary that a substernally implanted ICD like the ICD 5 is used. Rather, other ICDs would also be fully appropriate. Furthermore, other implantable pulse generators than the iLP 3 may be used for implementing the precedingly explained method.

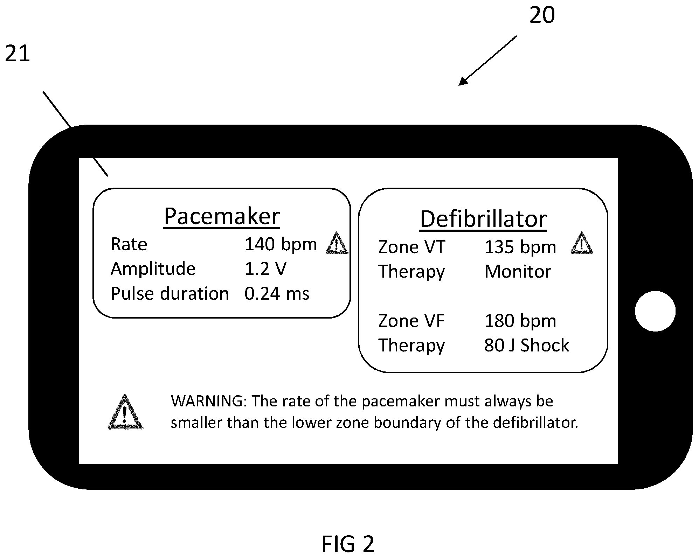

Figure 2 shows a smartphone 20 serving as control device for controlling the functioning of a cardiac rhythm management system like the system of iLP3 and ICD 5 shown in Figure 1. The smartphone 20 comprises a display 21 displaying specific information on operational parameters of a pacemaker as first implantable medical device and a defibrillator as second implantable medical device.

The operational parameters indicated for the pacemaker are the stimulation rate ("Rate"), the stimulation amplitude ("Amplitude") and the pulse duration ("Pulse duration"). In the present embodiment, the stimulation rate is set to 140 bpm.

The operational parameters for the defibrillator are the lower zone boundary for detecting a ventricular tachycardia ("Zone VT") and the therapy to be applied in such a case ("Therapy"). In the present embodiment, the lower zone boundary is set to 135 bpm. If such a cardiac frequency is detected by the defibrillator, no immediate countermeasure is to be taken. Rather, the cardiac state of the patient is to be monitored ("Therapy Monitor"). Thus, a shock delay of the defibrillator is activated.

Further operational parameters indicated for the defibrillator are the lower zone boundary for detecting a ventricular fibrillation ("Zone VF") and the therapy to be applied in case of exceeding this zone boundary ("Therapy"). The value zone VF is set in the present embodiment to 180 bpm. Thus, the first zone indicating a ventricular tachycardia ranges from 135 bpm to below 180 bpm. The second zone indicating a ventricular fibrillation encompasses cardiac rates of 180 bpm and higher.

If such a high cardiac rate indicating a ventricular fibrillation is detected by the defibrillator, a defibrillation shock with an energy of 80 J is applied ("Therapy 80 J Shock").

According to the presently described method, at least one operational parameter of the pacemaker is compared with at least one operational parameter of the defibrillator. In the present embodiment, the stimulation rate of the pacemaker is compared with the lower zone boundary ("Zone VT") of the defibrillator.

This comparison is made under application of a parameter conflict rule requiring that the stimulation frequency of the pacemaker needs to be lower than the lower zone boundary of the defibrillator. In the present case, this parameter conflict rule is not fulfilled. Therefore, a parameter conflict is detected. This parameter conflict as indicated by warning logos behind the chosen stimulation rate of the pacemaker and the chosen lower zone boundary of the defibrillator. In addition, the same warning logo is used together with explanatory text below the depiction of the operational parameters of the pacemaker and the defibrillator. In the present case, a user of the smartphone 20 is informed that the stimulation rate of the pacemaker must always be smaller than the lower zone boundary of the defibrillator. However, this condition is presently not fulfilled.

The smartphone 20 will therefore not transfer the chosen stimulation rate and the lower zone boundary to the pacemaker and the defibrillator. Rather, a correction of the input data is required so that the applied parameter conflict rule is fulfilled. Only afterwards, the chosen settings will be transferred to the respective implantable medical devices.

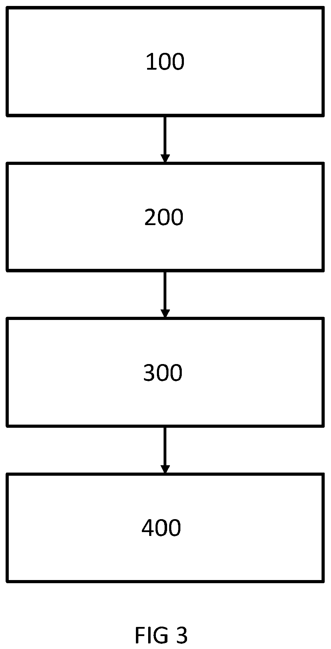

Figure 3 schematically depicts the sequence of an embodiment of the presently described method for controlling the operation of at least two implantable medical devices. In a first step 100, a first operational parameter (like the stimulation rate) of a first implantable medical device (like an implantable pulse generator) is received by a control device.

In a second method step 200, a second operational parameter (like a lower zone boundary for defibrillation) of a second implantable medical device (like an implantable cardioverter/defibrillator) is received with the same control device. Whereas the second step 200 is depicted in Figure 3 to be carried out after the first step 100, such a sequence is not necessary. Rather, the second step 200 can also be performed prior to or concomitantly with the first step 100.

Afterwards, a parameter conflict checking 300 takes place. For this purpose, it is checked whether there exists a conflict between the first operational parameter and the second operational parameter. Such a conflict is present if the received first operational parameter and the received second operational parameter would preclude a safe operation of the two implantable medical devices.

If such a conflict has been detected, the first operational parameter, the second operational parameter and data on the conflict will be displayed on the display of the control device in a displaying step 400. The user of the control device is then directly informed that the chosen operational parameters are in conflict to each other and require correction.

## Claims

1. Method for controlling the operation of at least two implantable medical devices (3, 5) for stimulating a human or animal heart (2), the method comprising the following steps:
a) receiving (100), with a control device (20), a first operational parameter of a first device (3) of the at least two implantable medical devices (3, 5);
b) receiving (200), with the control device (20), a second operational parameter of a second device (5) of the at least two implantable medical devices (3, 5);
c) automatically checking (300) whether there exists a conflict between the first operational parameter and the second operational parameter precluding a safe operation of the first device (3) and the second device (5); and
d) if a conflict has been identified, visualizing (400) the first operational parameter, the second operational parameter, and data on the conflict on a display (21) of the control device (20).

2. Method according to claim 1, **characterized in that** the control device (20) receives the first operational parameter and/or the second operational parameter from an input of a user of the control device (20).

3. Method according to claim 1 or 2, **characterized in that** the control device (20) receives the first operational parameter from a memory of the first device (3) and/or the second operational parameter from a memory of the second device (5).

4. Method according to any of the preceding claims, **characterized in that** the control device (20) prevents an application of the first parameter by the first device (3) and/or an application of the second parameter by the second device (5), if a conflict has been identified.

5. Method according to any of the preceding claims, **characterized in that** the first operational parameter is chosen from the group consisting of a stimulation frequency, frequency boundaries that need to be exceeded for delivering an antitachycardic pacing, and a condition to be fulfilled for delivery of an antitachycardic pacing.

6. Method according to any of the preceding claims, **characterized in that** the second operational parameter is chosen from the group consisting of a lower frequency boundary for defibrillation, an upper frequency boundary for defibrillation, a frequency zone for defibrillation between a lower and an upper frequency boundary, and an activation of a shock function of the second implantable medical device.

7. Method according to any of the preceding claims, **characterized in that** the first device is a leadless pacemaker (3).

8. Method according to claim 7, **characterized in that** the leadless pacemaker (3) comprises a function for an antitachycardic stimulation of a human or animal heart.

9. Method according to any of the preceding claims, **characterized in that** the second device is an implantable cardioverter/defibrillator (5).

10. Method according to claim 9, **characterized in that** the second device is a non-transvenously implantable cardioverter/defibrillator (5).

11. Method according to any of the preceding claims, **characterized in that** the first device (3) comprises a first patient identifier and the second device (5) comprises a second patient identifier, wherein the control device (20) queries the first patient identifier and the second patient identifier and performs the checking (300) whether there exists a conflict between the first operational parameter and the second operational parameter only if the first patient identifier and the second patient identifier are assigned to the same patient.

12. Method according to any of the preceding claims, **characterized in that** the method is carried out by at least one of a programming device application, a server application, an application for remote monitoring the at least two implantable medical devices, an application for remote programming the at least two implantable medical devices, and a mobile app.

13. Cardiac rhythm management system, comprising a first implantable medical device (3) for stimulating a human or animal heart (2), a second implantable medical device (5) for stimulating the same heart (2), and a control device (20) operatively coupled to the first implantable medical device (3) and the second implantable medical device (5), wherein the control device (20) comprises a memory and a processor, wherein the memory comprises computer-readable code that causes the processor to perform the following steps when executed on the processor:
a) receiving (100), with the control device (20), a first operational parameter of the first implantable medical device (3);
b) receiving (200), with the control device (20), a second operational parameter of the second implantable medical device (5);
c) automatically checking (300) whether there exists a conflict between the first operational parameter and the second operational parameter precluding a safe operation of the first implantable medical device (3) and the second implantable medical device (5); and
d) if a conflict has been identified, visualizing (400) the first operational parameter, the second operational parameter, and data on the conflict on a display (21) of the control device (20).

14. Cardiac rhythm management system according to claim 13, **characterized in that** the control device (20) is operatively coupled to the first implantable medical device (3) and the second implantable medical device (5) in a wireless manner.

15. Computer program product comprising computer-readable code that causes the processor of the cardiac rhythm management system of any of claims 13-14 perform the following steps when executed on the processor:
a) receiving (100) a first operational parameter of a first implantable medical device (3);
b) receiving (200) a second operational parameter of a second implantable medical device (5);
c) automatically checking (300) whether there exists a conflict between the first operational parameter and the second operational parameter precluding a safe operation of the first implantable medical device (3) and the second implantable medical device (5); and
d) if a conflict has been identified, visualizing (400) the first operational parameter, the second operational parameter, and data on the conflict on a display (21).

## Patentansprüche

1. Verfahren zur Steuerung des Betriebs von mindestens zwei implantierbaren medizinischen Geräten (3, 5) zur Stimulation eines menschlichen oder tierischen Herzens (2), wobei das Verfahren die folgenden Schritte umfasst:
a) Empfangen (100) eines ersten Betriebsparameters eines ersten Geräts (3) der mindestens zwei implantierbaren medizinischen Geräte (3, 5) mit einer Steuervorrichtung (20);
b) Empfangen (200) eines zweiten Betriebsparameters einer zweiten Vorrichtung (5) der mindestens zwei implantierbaren medizinischen Vorrichtungen (3, 5) mit der Steuervorrichtung (20);
c) automatisches Überprüfen (300), ob ein Konflikt zwischen dem ersten Betriebsparameter und dem zweiten Betriebsparameter vorliegt, der einen sicheren Betrieb der ersten Vorrichtung (3) und der zweiten Vorrichtung (5) ausschließt; und
d) wenn ein Konflikt festgestellt wurde, Visualisieren (400) des ersten Betriebsparameters, des zweiten Betriebsparameters und von Daten zu dem Konflikt auf einer Anzeige (21) der Steuervorrichtung (20).

2. Verfahren nach Anspruch 1, **dadurch gekennzeichnet, dass** die Steuervorrichtung (20) den ersten Betriebsparameter und/oder den zweiten Betriebsparameter von einer Eingabe eines Benutzers der Steuervorrichtung (20) empfängt.

3. Verfahren nach Anspruch 1 oder 2, **dadurch gekennzeichnet, dass** die Steuervorrichtung (20) den ersten Betriebsparameter von einem Speicher der ersten Vorrichtung (3) und/oder den zweiten Betriebsparameter von einem Speicher der zweiten Vorrichtung (5) empfängt.

4. Verfahren nach einem der vorstehenden Ansprüche, **dadurch gekennzeichnet, dass** die Steuervorrichtung (20) eine Anwendung des ersten Parameters durch die erste Vorrichtung (3) und/oder eine Anwendung des zweiten Parameters durch die zweite Vorrichtung (5) verhindert, wenn ein Konflikt festgestellt wurde.

5. Verfahren nach einem der vorstehenden Ansprüche, **dadurch gekennzeichnet, dass** der erste Betriebsparameter aus der Gruppe ausgewählt wird, die aus einer Stimulationsfrequenz, Frequenzgrenzen, die für die Abgabe einer antitachykarden Stimulation überschritten werden müssen, und einer Bedingung, die für die Abgabe einer antitachykarden Stimulation erfüllt sein muss, besteht.

6. Verfahren nach einem der vorstehenden Ansprüche, **dadurch gekennzeichnet, dass** der zweite Betriebsparameter aus der Gruppe ausgewählt wird, die aus einer unteren Frequenzgrenze für die Defibrillation, einer oberen Frequenzgrenze für die Defibrillation, einer Frequenzzone für die Defibrillation zwischen einer unteren und einer oberen Frequenzgrenze und einer Aktivierung einer Schockfunktion des zweiten implantierbaren medizinischen Geräts besteht.

7. Verfahren nach einem der vorstehenden Ansprüche, **dadurch gekennzeichnet, dass** das erste Gerät ein kabelloser Herzschrittmacher (3) ist.

8. Verfahren nach Anspruch 7, **dadurch gekennzeichnet, dass** der kabellose Herzschrittmacher (3) eine Funktion zur antitachykarden Stimulation eines menschlichen oder tierischen Herzens umfasst.

9. Verfahren nach einem der vorstehenden Ansprüche, **dadurch gekennzeichnet, dass** das zweite Gerät ein implantierbarer Kardioverter/Defibrillator (5) ist.

10. Verfahren nach Anspruch 9, **dadurch gekennzeichnet, dass** das zweite Gerät ein nicht-transvenös implantierbarer Kardioverter/Defibrillator (5) ist.

11. Verfahren nach einem der vorstehenden Ansprüche, **dadurch gekennzeichnet, dass** die erste Vorrichtung (3) eine erste Patientenidentifikation und die zweite Vorrichtung (5) eine zweite Patientenidentifikation umfasst, wobei die Steuervorrichtung (20) die erste Patientenidentifikation und die zweite Patientenidentifikation abfragt und die Überprüfung (300) durchführt, ob ein Konflikt zwischen dem ersten Betriebsparameter und dem zweiten Betriebsparameter besteht, nur wenn die erste Patienten-Kennung und die zweite Patienten-Kennung demselben Patienten zugeordnet sind.

12. Verfahren nach einem der vorstehenden Ansprüche, **dadurch gekennzeichnet, dass** das Verfahren durch mindestens eine der folgenden Anwendungen durchgeführt wird: eine Programmiergeräteanwendung, eine Serveranwendung, eine Anwendung zur Fernüberwachung der mindestens zwei implantierbaren medizinischen Geräte, eine Anwendung zur Fernprogrammierung der mindestens zwei implantierbaren medizinischen Geräte und eine mobile App.

13. Herzrhythmus-Managementsystem, umfassend ein erstes implantierbares medizinisches Gerät (3) zur Stimulation eines menschlichen oder tierischen Herzens (2), einem zweiten implantierbaren medizinischen Gerät (5) zur Stimulation desselben Herzens (2) und einer Steuervorrichtung (20), die funktionsfähig mit dem ersten implantierbaren medizinischen Gerät (3) und dem zweiten implantierbaren medizinischen Gerät (5) gekoppelt ist, wobei die Steuervorrichtung (20) einen Speicher und einen Prozessor umfasst, wobei der Speicher einen computerlesbaren Code umfasst, der den Prozessor veranlasst, die folgenden Schritte auszuführen, wenn er auf dem Prozessor ausgeführt wird:
a) Empfangen (100) eines ersten Betriebsparameters des ersten implantierbaren medizinischen Geräts (3) mit der Steuervorrichtung (20);
b) Empfangen (200) eines zweiten Betriebsparameters des zweiten implantierbaren medizinischen Geräts (5) mit der Steuervorrichtung (20);
c) automatisches Überprüfen (300), ob ein Konflikt zwischen dem ersten Betriebsparameter und dem zweiten Betriebsparameter besteht, der einen sicheren Betrieb des ersten implantierbaren medizinischen Geräts (3) und des zweiten implantierbaren medizinischen Geräts (5) verhindert; und
d) wenn ein Konflikt festgestellt wurde, Visualisieren (400) des ersten Betriebsparameters, des zweiten Betriebsparameters und der Daten über den Konflikt auf einer Anzeige (21) der Steuervorrichtung (20).

14. Herzrhythmus-Managementsystem nach Anspruch 13, **dadurch gekennzeichnet, dass** die Steuervorrichtung (20) drahtlos mit dem ersten implantierbaren medizinischen Gerät (3) und dem zweiten implantierbaren medizinischen Gerät (5) verbunden ist.

15. Computerprogrammprodukt, das einen computerlesbaren Code umfasst, der bewirkt, dass
der Prozessor des Herzrhythmus-Managementsystems gemäß einem der Ansprüche 13 bis 14 bei Ausführung auf dem Prozessor die folgenden Schritte ausführt:
a) Empfangen (100) eines ersten Betriebsparameters eines ersten implantierbaren medizinischen Geräts (3);
b) Empfangen (200) eines zweiten Betriebsparameters eines zweiten implantierbaren medizinischen Geräts (5);
c) automatisches Überprüfen (300), ob ein Konflikt zwischen dem ersten Betriebsparameter und dem zweiten Betriebsparameter besteht, der einen sicheren Betrieb des ersten implantierbaren medizinischen Geräts (3) und des zweiten implantierbaren medizinischen Geräts (5) verhindert; und
d) wenn ein Konflikt festgestellt wurde, Visualisieren (400) des ersten Betriebsparameters, des zweiten Betriebsparameters und der Daten zum Konflikt auf einem Display (21).

## Revendications

1. Procédé de commande du fonctionnement d'au moins deux dispositifs médicaux implantables (3, 5) destinés à stimuler un cœur humain ou animal (2), le procédé comprenant les étapes suivantes consistant à :
a) recevoir (100), avec un dispositif de commande (20), un premier paramètre de fonctionnement d'un premier dispositif (3) parmi les au moins deux dispositifs médicaux implantables (3, 5) ;
b) recevoir (200), avec le dispositif de commande (20), un second paramètre de fonctionnement d'un second dispositif (5) parmi les au moins deux dispositifs médicaux implantables (3, 5) ;
c) vérifier automatiquement (300) s'il existe un conflit entre le premier paramètre de fonctionnement et le second paramètre de fonctionnement empêchant un fonctionnement en toute sécurité du premier dispositif (3) et du second dispositif (5) ; et
d) si un conflit a été identifié, visualiser (400) le premier paramètre de fonctionnement, le second paramètre de fonctionnement et les données relatives au conflit sur un affichage (21) du dispositif de commande (20).

2. Procédé selon la revendication 1, **caractérisé en ce que** le dispositif de commande (20) reçoit le premier paramètre de fonctionnement et/ou le second paramètre de fonctionnement à partir d'une entrée d'un utilisateur du dispositif de commande (20).

3. Procédé selon la revendication 1 ou 2, **caractérisé en ce que** le dispositif de commande (20) reçoit le premier paramètre de fonctionnement depuis une mémoire du premier dispositif (3) et/ou le second paramètre de fonctionnement depuis une mémoire du second dispositif (5).

4. Procédé selon l'une quelconque des revendications précédentes, **caractérisé en ce que** le dispositif de commande (20) empêche une application du premier paramètre par le premier dispositif (3) et/ou une application du second paramètre par le second dispositif (5), si un conflit a été identifié.

5. Procédé selon l'une quelconque des revendications précédentes, **caractérisé en ce que** le premier paramètre de fonctionnement est choisi dans le groupe constitué par une fréquence de stimulation, des limites de fréquence qui doivent être dépassées pour administrer une stimulation antitachycardique, et une condition à remplir pour l'administration d'une stimulation antitachycardique.

6. Procédé selon une quelconque des revendications précédentes, **caractérisé en ce que** le second paramètre de fonctionnement et choisi dans le groupe constitué par une limite de fréquence inférieure pour la défibrillation, une limite de fréquence supérieure pour la défibrillation, une zone de fréquence pour la défibrillation entre des limites de fréquence inférieure et supérieure, et une activation d'une fonction de choc du second dispositif médical implantable.

7. Procédé selon l'une quelconque des revendications précédentes, **caractérisé en ce que** le premier dispositif est un stimulateur cardiaque sans câbles (3).

8. Procédé selon la revendication 7, **caractérisé en ce que** le stimulateur cardiaque sans câbles (3) comprend une fonction pour une stimulation antitachycardique d'un cœur humain ou animal.

9. Procédé selon l'une quelconque des revendications précédentes, **caractérisé en ce que** le second dispositif est un défibrillateur automatique implantable (5).

10. Procédé selon la revendication 9, **caractérisé en ce que** le second dispositif est un défibrillateur automatique implantable (5) de manière non transveineuse.

11. Procédé selon l'une quelconque des revendications précédentes, **caractérisé en ce que** le premier dispositif (3) comprend un premier identifiant de patient et le second dispositif (5) comprend un second identifiant de patient, dans lequel le dispositif de commande (20) demande le premier identifiant de patient et le second identifiant de patient et réalise l'étape consistant à vérifier (300) s'il existe un conflit entre le premier paramètre de fonctionnement et le second paramètre de fonctionnement uniquement si le premier identifiant de patient et le second identifiant de patient sont attribués au même patient.

12. Procédé selon l'une quelconque des revendications précédentes, **caractérisé en ce que** le procédé est mis en œuvre par au moins un parmi une application de dispositif de programmation, une application de serveur, une application destinée à surveiller à distance les au moins deux dispositifs médicaux implantables, une application destinée à programmer à distance les au moins deux dispositifs médicaux implantables et une application mobile.

13. Système de gestion du rythme cardiaque, comprenant un premier dispositif médical implantable (3) destiné à stimuler un cœur humain ou animal (2), un second dispositif médical implantable (5) destiné à stimuler le même cœur (2), et un dispositif de commande (20) couplé de manière fonctionnelle au premier dispositif médical implantable (3) et au second dispositif médical implantable (5), dans lequel le dispositif de commande (20) comprend une mémoire et un processeur, dans lequel la mémoire comprend un code lisible par ordinateur qui amène le processeur à réaliser les étapes suivantes lorsqu'elles sont exécutées sur le processeur :
a) recevoir (100), avec le dispositif de commande (20), un premier paramètre de fonctionnement du premier dispositif médical implantable (3) ;
b) recevoir (200), avec le dispositif de commande (20), un second paramètre de fonctionnement du second dispositif médical implantable (5) ;
c) vérifier automatiquement (300) s'il existe un conflit entre le premier paramètre de fonctionnement et le second paramètre de fonctionnement empêchant un fonctionnement en toute sécurité du premier dispositif médical implantable (3) et du second dispositif médical implantable (5) ; et
d) si un conflit a été identifié, visualiser (400) le premier paramètre de fonctionnement, le second paramètre de fonctionnement et les données relatives au conflit sur un affichage (21) du dispositif de commande (20).

14. Système de gestion du rythme cardiaque selon la revendication 13, **caractérisé en ce que** le dispositif de commande (20) est couplé de manière fonctionnelle au premier dispositif médical implantable (3) et au second dispositif médical implantable (5) d'une manière sans fil.

15. Produit de programme informatique comprenant un code lisible par ordinateur qui amène le processeur du système de gestion du rythme cardiaque selon l'une quelconque des revendications 13 à 14 à réaliser l'étape suivante lorsqu'il est exécuté sur le processeur :
a) recevoir (100) un premier paramètre de fonctionnement d'un premier dispositif médical implantable (3) ;
b) recevoir (200) un second paramètre de fonctionnement d'un second dispositif médical implantable (5) ;
c) vérifier automatiquement (300) s'il existe un conflit entre le premier paramètre de fonctionnement et le second paramètre de fonctionnement empêchant un fonctionnement en toute sécurité du premier dispositif médical implantable (3) et du second dispositif médical implantable (5) ; et
d) si un conflit a été identifié, visualiser (400) le premier paramètre de fonctionnement, le second paramètre de fonctionnement et les données relatives au conflit sur un affichage (21).
